# EUROPEAN PATENT SPECIFICATION

(11) **EP 1 637 135 B1**
(45) Date of publication and mention of the grant of the patent: **21.05.2008**
(21) Application number: 05255734.5
(22) Date of filing: 15.09.2005
(51) Int. Cl.: A61K 31/203, A61K 31/10, A61P 17/00, A61K 8/30

(54) **Compositions comprising dimethylsulfoxide and tretinoin for treating skin disorders**
Zusammensetzungen Dimethylsulfoxid und Tretinoin enhaltend zur Behandlung von Hautstörungen
Compositions comprenant du diméthylsulfoxyde et de la trétinoine pour le traitement des désordres de la peau

(30) Priority: 15.09.2004 US 609876
(43) Date of publication of application: 22.03.2006
(73) Proprietor: Aebi, Albert, 8002 Zurich (CH)
(72) Inventor: Aebi, Albert, 8002 Zurich (CH)
(74) Representative: Marsden, John Christopher

(56) References cited:
- WO-A-88/05653
- US-B1- 6 485 950

## Description

### CROSS-REFERENCE TO RELATED APPLICATION

This application claims benefit from U.S. Provisional Application Serial No. 60/609,876, filed September 15, 2004.

### Field of the Invention

The present invention relates generally to compositions for treatment of skin conditions, more particularly for treatment of skin disorders based on disturbed or disrupted gene expression where DNA damage is responsible for the skin condition. These are referred to hereinafter as conditions resulting from genetic alteration, genetic misformation and/or genetic abnormality.

### Background of the Invention

Dermatology encompasses, among other things, the vast field of the changeability of human skin. Illnesses or diseases of the skin generally can be divided into two main groups, namely, infections and genetic alterations.

Worldwide, the pharmaceutical and (in particular) cosmetic industries have developed and marketed numerous products to address the desires of those who wish to disguise certain appearances caused primarily by advanced age and light when medical treatment is ineffective. Age spots, skin folds, and non-infectious cellulites are among such appearances sought to be disguised.

### Age spots

The skin can develop different shades of pigmentation ranging from clear white to dark brown according to the intensity of light and UV-radiation in the various particular geographical regions of the earth. This is regulated by RNA-codes that have been developed over 100,000's of years by radiation from the sun that ranges in intensity from weak to strong. Melatonin, or derivatives thereof called Melatonoids, are used by the code of the skin cell to dye the skin into the various well-known shades to protect it against light.

When the code is irreparably damaged through additional excessive irritation by light, chemicals, or certain pressure on particular areas of the skin, dark brown areas or spots develop. This phenomenon is not strictly a question of age, but rather of duration and strength of light intensity and/or chemical damage. Age spots occur frequently among people without distinguishing significantly between age, education, or income. Thus, exogenous influences and irreparable damage to the DNA and RNA in the cell code lead frequently to skin spots, also referred to as "age spots." Such changes are profound and none of the many commercially available products exhibits any satisfactory influence or shows any activity for successful treatment.

### Cellulites

Cellulites (non-infection type) is also known as dermopanniculosis deformans. In this type of cellulites, there is formation of a visible change in the skin, which occurs mainly on the skin of the upper legs, upper arms and sometimes part of the skin of the abdomen, and lower part of the bottom. This formation is caused by a change in the subcutis-structure concerning primarily the septes of the fatty tissue of the subcutis, resulting in fatty cells collecting in the corium, which follows the epidermis as the second large skin layer. An external view reveals lumpy irregularities, or in its finer form, "orange skin," whose irregularities are not inflamed processes or changes.

This type of cellulites is different than the infectious cellulite in the skin caused mainly by Streptococcus pyrogenes or Staphylococcus aureus. Cellulite by infection is rare compared to the non-infectious cellulite described above and must be treated in a completely different manner, using antibiotics. Moreover, infectious cellulites does not form the above-noted obvious skin changes seen in the non-infectious type. Disturbed cell codes resulting primarily from age can also cause skin folds, for which treatment is also desired.

No genuinely effective remedy has yet been discovered which adequately addresses the above conditions. Specifically, no remedy has yet been found in which age spots or cellulites become influenced or disappear completely and/or a new and normal skin develops within a relatively short time and wherein the new appearance can be maintained in a healthy condition.

Many tretinoin-containing formulations have been proposed for treatment for photodamaged skin conditions, but none has yet been found particularly effective. The following represent examples of such known formulations that are sometimes prescribed. Patients, however, are often dissatisfied and discontinue their use:
1. Tretinoin fabricated by Louis Widmer in Switzerland

| | |
|---|---|
| Composition: | Tretinoin |
| | Urea |
| | Triclosan |
| | |
| Indication: | Topic infection, Acne classified as antibiotic |

2. Retin A Cream fabricated by Janssen-Cilag in Switzerland

| | |
|---|---|
| Composition: | Tretinoin, Cream 0.025% and 0.05% |
| | |
| Indication: | Treatment of Acne |

U.S. Patent No. 6,485,950 cross-refers to U.S. Patent No. 5,470,876 as disclosing formulation of superoxide dismutase as an anti-alopecia agent in a topical pharmaceutical composition in the form of a lotion comprising, *inter alia,* dimethylsulfoxide, tretinoin and the hydrophilic ointment Dermovan.

WO 88/05653 discloses a hair growth-stimulating topical composition likewise containing dimethylsulfoxide, tretinoin and Dermovan.

There remains a need in the art for an effective remedy that overcomes the above and other problems seen in the art with respect to treatment of photodamaged and age-related skin conditions. Specifically, there is a need for novel compositions that are effective in treating such skin conditions, particularly those conditions resulting from genetic alteration, genetic misformation, and/or genetic abnormality, by repairing the damaged DNA in skin cells that leads to these and other conditions.

### SUMMARY OF THE INVENTION

The present invention provides compositions for the treatment of skin conditions resulting from genetic alteration, genetic misformation and/or genetic abnormality. Such skin conditions include, without limitation, age spots, liver spots, photodamaged skin, skin folds, diminished hair growth, and cellulite not caused by infection, which presents itself as "orange skin" and/or in the form of cellulite exhibiting finer or larger lumpy irregularities. To overcome the problems associated with previously reported compositions for the treatment of such skin conditions, the present invention provides novel compositions. In an aspect of the invention, compositions are provided which are capable through topical skin application, of rapidly delivering a substance to an area of DNA damage responsible for a skin condition, and repairing the damaged DNA, thus treating the condition. More specifically, in an aspect, the substance is delivered directly to the skin cell for repair of the damaged DNA.

Compositions in accordance with the present invention include tretinoin within a range of 0.01-1 part, and dimethylsulfoxide (DMSO) within a range of 1-45 parts. The compositions also include urea within a range of 5-40 parts and/or L-carnitin base or L-carnitin hydrochloride within a range of 5-15 parts. In some embodiments, additional ingredients may comprise a balance of about 50-130 parts, which may include a pharmaceutically or cosmetically acceptable carrier, e.g. a hydrophilic nonionic carrier within a range of about 45-90 parts, which may be, for example, an ointment, lotion or spray carrier.

Some preferred embodiments include about 20-40 parts urea. Some preferred embodiments include 45-75 parts carrier.

Alternatively, compounds in accordance with the invention may include urea within a range of about 5-40 parts, tretinoin within a range of about 0.01-1 part, dimethylsulfoxide within a range of about 1-45 parts and a carrier such as pharmaceutically or cosmetically acceptable solvent within a range of about 45-90 parts. Particularly preferred compositions for treatment of non-infectious cellulite further include L-carnitin base or L-carnitin hydrochloride within a range of about 5-15 parts.

Unless otherwise indicated, in all of the formulations described herein, parts indicated in the composition are by weight.

Preferred compositions are topical formulations. Suitable formulations may include, without limitation, ointments, lotions, sprays, and any other suitable topical formulations that achieve the desired result. The invention additionally provides use of tretinoin and dimethylsulfoxide in respective amounts of 0.01-1 part and 1-45 parts, optionally together with urea in an amount of 5-40 parts and optionally further together with L-carnitin base or L-carnitin hydrochloride in an amount of 5-15 parts, for the manufacture of a medicament for treatment of a skin condition which is an age spot, liver spot, photodamaged skin or a skin fold.

Moreover the invention provides a method of cosmetically treating a skin condition, e.g. non-infection cellulite, comprising administering a composition as hereinbefore defined to an area of skin condition to be treated.

### DETAILED DESCRIPTION OF THE INVENTION

The present invention relates to treatment of skin conditions resulting from genetic alteration, genetic misformation and/or genetic abnormality. Such skin conditions include, without limitation, age spots, liver spots, photodamaged skin, skin folds, diminished hair growth, and cellulite not caused by infection, which presents itself as "orange skin" and/or in the form of cellulite exhibiting finer or larger lumpy irregularities. The compositions of the invention permit repair of damaged DNA by administration of tretinoin, which is quickly delivered to the area of damaged DNA in the cell by DMSO (i.e., to nuclear retinoic acid receptors, RAR). In preferred embodiments, this mechanism is facilitated with urea in the composition, which supports water regulation in the skin, and in some embodiments, with carnitin, which supports fatty acid regulation and degradation in the skin cell. This new approach of delivering tretinoin to the source of the skin disorders (i.e., in the DNA code), results in a fast disappearance of age/light spots and a visible improvement in cellulites. During preclinical tests of the inventive compositions in the treatment of skin spots, a remarkable, definite improvement in skin folds was also observed. The folds became less deep, smoother, and some disappeared completely. Such folds also result from disturbed cell DNA codes caused primarily by age.

The compositions of the present invention are suitable for use in the treatment of all skin disorders that are based on disturbed or disrupted gene expression. In addition to those mentioned above, such disorders also include, *inter alia,* basal cell carcinoma and melanoma. In preferred embodiments, the compositions of the present invention comprise a unique combination of urea, tretinoin, DMSO and a hydrophilic nonionic carrier, such as an ointment carrier. Alternate preferred embodiments comprise a unique combination of urea, tretinoin, DMSO, and a solvent suitable for formulation into a lotion or spray. Further alternate embodiments include carnitin. The methods of the present invention provide for treatment of various skin disorders through the administration of one or more of the inventive formulations.

In one embodiment, the invention includes compositions for treatment of skin conditions, comprising 20 to 40 parts by weight urea, 0.01 to 1 part by weight tretinoin, 1 to 45 parts by weight dimethylsulfoxide and 45 to 75 parts by weight hydrophilic nonionic ointment. Particularly preferred compositions for treatment of non-infectious cellulite further include L-carnitin base or L-carnitin hydrochloride within a range of 5 to 15 parts. The compositions described herein are preferably for topical administration. Such topical formulations can be readily prepared by techniques known to those of ordinary skill in the art.

The hydrophilic nonionic ointment preferably comprises polysorbate 60 within a range of 1 to 10 parts, cetylstearylalcohols within a range of 5 to 15 parts, glycerol (such as glycerol 85%) within a range of 5 to 15 parts, petroleum jelly such as white vaseline within a range of 20 to 30 parts, and water within a range of 1 to 10 parts.

One particularly preferred composition of the hydrophilic nonionic ointment component includes about 5 parts by weight polysorbate 60, about 10 parts by weight cetylstearylalcohols, about 10 parts by weight glycerol 85%, about 25 parts by weight pertroleum jelly and about 5 parts by weight water.

One particularly preferred embodiment for treatment of age spots and skin folds includes about 30 parts by weight urea, about 0.05 parts by weight tretinoin, about 5 parts by weight dimethylsulfoxide and about 65 parts by weight hydrophilic nonionic ointment.

One particularly preferred composition for treatment of non-infectious cellulite includes about 30 parts by weight urea, about 10 parts L-carnitin base or L-carnitin hydrochloride, about 0.05 parts by weight tretinoin, about 5 parts by weight dimethylsulfoxide and about 55 parts by weight hydrophilic nonionic ointment.

Alternate embodiments of the invention may include urea within a range of 5-40 parts, tretinoin within a range of 0.01-1 part, dimethylsulfoxide within a range of 1-45 parts and a solvent within a range of 45-90 parts. The solvent is preferably suitable for formulating the composition into a lotion or spray. Particularly preferred compositions for treatment of non-infectious cellulite further include L-carnitin base or L-carnitin hydrochloride within a range of 5 to 15 parts. These compositions are preferably for topical administration. Such topical formulations can be readily prepared by techniques known to those of ordinary skill in the art.

The solvent preferably comprises isopropyl alcohol (2-propanol, isopropanol) and water. In particularly preferred embodiments, the alcohol is present in the solvent in an amount that is about 70 parts per volume and the water is present in an amount that is about 30 parts per volume.

One particularly preferred embodiment for treatment of age spots and skin folds includes about 10 parts by weight urea, about 0.05 parts by weight tretinoin, about 10 parts by weight dimethylsulfoxide and about 80 parts by weight of solvent suitable for formulating the composition into a lotion or spray.

One particularly preferred composition for treatment of non-infectious cellulite includes about 10 parts by weight urea, about 10 parts L-carnitin base or L-carnitin hydrochloride, about 0.05 parts by weight tretinoin, about 10 parts by weight dimethylsulfoxide and about 70 parts by weight solvent suitable for formulating into a lotion or spray.

The compositions described herein may be applied to an area of skin affected by the skin condition to be treated. Effective amounts generally are within a range of about 0.001-10 gm, preferably within a range of 0.01-1 gm.

In preferred embodiments, the skin condition results from genetic alteration, genetic misformation, genetic abnormality or a combination thereof. In particular, the skin condition is an age spot, a liver spot, photodamaged skin, a skin fold, or diminished hair growth. As described above, compositions containing L-carnitin are particularly suitable for use in methods of treating non-infectious cellulite which include administering the composition to an area of skin with non-infectious cellulite. Treating includes slowing, stopping, or reversing the condition's progression. Thus, for example, the methods described herein can be used to fade skin spots, repair photodamaged skin, diminish or eliminate skin folds and non-infectious cellulite, enhance hair growth, or otherwise slow, stop or reverse the progression of the various skin conditions described herein. One of ordinary skill in the art can readily determine the optimal dosing schedules to achieve the desired results described herein.

Due to possible variations in entry mechanisms into the skin of different galenic forms (i.e., ointments, lotions, sprays, etc.), more than one of the formulations described herein may be applied in combination or in sequence. Such applications may include, for example, administering an ointment formulation followed by a lotion or spray formulation.

### Descriptions of the individual active ingredients present in the inventive compositions

### Urea

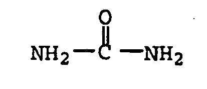

Urea is a physiological regulator of nitrogen excretion in mammals. It is synthesized in the liver as an end-product of protein catabolism and degradation and is excreted in urine.

Urea also occurs naturally in skin and is an osmotic agent similar to mannitol. It is applied topically in the treatment of ichthyosis (fish-skin) and hyperkeratotic skin disorders. When applied topically, urea has hydrating and keratolytic properties. In the management of ichthyosis and other dry skin disorders, urea is applied in creams or lotions typically containing 10% to 25% of the ingredient.

As an endproduct of protein-metabolism in the urea-cycle, about 30 g urea are formed daily. When large amounts of proteins are used in nutrition, this amount can rise 2-3 times per day.

In addition to ichthyosis, urea is also used in ointments and creams for the treatment of wounds.

### L - Carnitin Base and L - Carnitin Hydrochloride

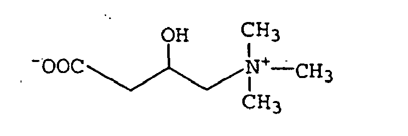

### L-Carnitin Base, the inner salt

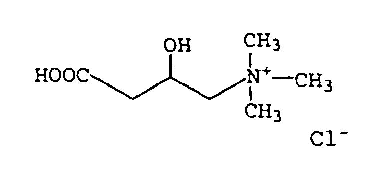

### L-Carnitin Hydrochloride, gastric and pancreatic secretion stimulant

L-carnitin is also known as γ-Trimethyl-β-hydroxybutyrobetain (Vitamin B_{T}) and is an essential cofactor of fatty acid metabolism. It is also an antihyperlipoproteinemic and a carrier for acyl-function in the membrane of the mitochondrium. Its therapeutic use in primary and secondary carnitine deficiencies relates to its role in fatty acid metabolism. Longstanding leg ulcers respond dramatically to carnitine therapy and carnitive supplementation plays a cardioprotective role in cases of cardiomyopathies, and causes reduction of infarct size and prevention of arrhythmia.

No side effects were observed in children when dosage intakes of 200 - 800 mg/kg of carnitin were given.

### Tretinoin

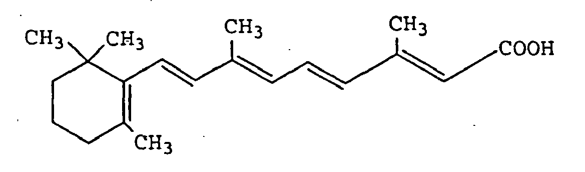

all-trans retinoic acid
Vitamin A acid
Retin - A
Retinova

Tretinoin is a physiological metabolite of Vitamin A. It affects gene expression via nuclear retinoic acid receptors (RAR) and mediates cellular growth and differentiation. Tretinoin is useful in the treatment of Chloasma (also known as Melasma), which is acquired Hypermelanosis of parts of skin through sunlight exposure. Tretinoin is also often used for topical and/or peroral therapy of acne vulgaris.

### Tretinoin Interaction

Tretinoin is metabolized by microsomal liver enzymes. Therefore, there is a potential for interaction between oral tretinoin and inhibitors or inducers of these enzymes.

### Pharmacokinetics

Following oral administration, tretinoin is well absorbed from the gastrointestinal tract and peak plasma concentrations are obtained after 1-2 hours. Oral bioavailability is about 50%. Tretinoin is highly bound to plasma proteins and undergoes metabolism in the liver. Metabolites include isotretinoin, 4-oxo-trans retinoic acid, and 4-oxo-cis retinoic acid.

The terminal elimination half-life of tretinoin is 0.5 to 2 hours and tretinoin is excreted in the bile and the urine.

### Common uses and administration of tretinoin

Tretinoin, a retinoid and the acid form of vitamin A is used primarily in the topical treatment of acne vulgaris in which comedones, papules and pustules predominate.

### Neoplastic disorders

A number of retinoids have been shown to act against leukemic cells. Some beneficial effects have been reported in the treatment of actinic keratoses, basal cell carcinoma, metastatic melanoma, and dysplastic naevi.

### Skin disorders

Among the vast number of uses of tretinoin, applications and administration in the treatment of skin disorders so far have been of minor importance, characterized by variable success and transient effect only. A number of studies suggest that tretinoin can reverse some of the skin changes associated with chronic exposure to sunlight. However, the effects seen during treatment have been transient, with the skin reverting to its pretreatment state once application stops. One study has found that several changes taken as indicative of an anti-photo-aging effect, such as thickening of the skin and epidermis and an increase in the blood supply, were not specific to topically applied retinoids and could equally have been produced by the use of an abrasive preparation.

### Dimethyl Sulfoxide (DMSO)

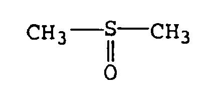

Sulphinyl-bis-methane
Dimethylis Sulfoxidum
Methylsulfoxide
melting point: 18.3° C

DMSO is rapidly absorbed through skin and mucous membranes. It enhances dermal absorption of many other chemicals.

DMSO is a solvent for many organic compounds, including fats and carbohydrates. It is typically used in the treatment of scleroderma. When topically applied, DMSO reduces swelling due to trauma. DMSO is also locally used as an antiseptic and antiphlogistic.

Systemic effects of DMSO, such as gastrointestinal disturbances, drowsiness, headache, and hypersensitive reaction may occur after administration of higher amounts by any route.

A Swiss preparation known as Sportusal^{™} is often used in sports applications. According to a recent literature search, there appear to be no serious complaints of side effects (DMSO concentration of 5%). DMSO, when used as a penetrating base for other drugs after topical application, may enhance these drugs' activities.

### Pharmacokinetics

DMSO is readily absorbed after administration by all routes and is metabolized by oxidation to dimethylsulfone and by reduction to dimethylsulfide.

DMSO and the sulfone metablite are excreted in the urine and feces. Dimethylsulfide is excreted through the lungs and skin and is responsible for a characteristic odor in the breath.

### Uses and administration

DMSO is a highly polar substance which has exceptional solvent properties for both organic and inorganic chemicals. It is widely used as an industrial solvent. It also has been reported to have a wide spectrum of pharmacological activity, including membrane penetration, anti-inflammatory effects, local analgesia, weak bacteriostasis, diuresis, vasodilatation, dissolution of collagen, and free radical scavenging.

The principal use of DMSO is as a vehicle for drugs, as it aids penetration of the drug into the skin.

DMSO has been administered orally, intravenously, or topically for a wide range of particular indications, including cutaneous and musculoskeletal disorders. However, evidence of beneficial effect is limited.

Interstitial cystitis is an inflammatory condition of the bladder of unknown etiology. Symptoms include pain, urinary frequency, urinary urgency, and nocturia. Bladder instillations of a 50% aqueous solution of DMSO appear to alleviate symptoms. The chief effect of DMSO may be on sensory nerves since there appears to be no significant alteration in the endoscopic or morphological appearance of the bladder after such treatment. Treatment has successfully been repeated ad relapse.

### Hydrophilic non ionic ointment

A classical basis ointment composition is used in some embodiments of the present invention, which ointment can be found as a formula in Deutsches Arzneimittel Buch and Pharmacopoe Helveticae.

This ointment is commercially available in any amount. This basic ointment is white, practically without odor, and easy to remove from the skin by washing with water. It can easily be preserved by including 0.1% sorbic acid in its composition.

As an oil-in-water cream, this basic ointment is galenically flawless. Its common use for many purposes is well known in the art as seen in the available literature noted above.

### Solvent

### Isopropyl alcohol

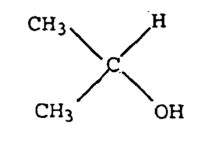

Isopropyl alcohol is a clear, colorless, mobile, volatile, flammable liquid with a characteristic spirituous odor, and is miscible with water.

The World Heath Organization (WHO) has indicated that for disinfection of living tissues isopropyl alcohol 70% is effective.

Isopropyl alcohol is a known antiseptic with bactericidal properties similar to ethanol and is used for protective skin cleansing and as an ingredient in preparations for disinfection of hands and surfaces.

Isopropyl alcohol also has been used as a solvent, particularly in cosmetics and perfumes, and as a vehicle for other compounds. There appears to be little resorption through the skin.

### Substances similar to Dimethylsulfoxide

### D-Mannitol

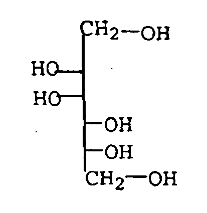

Mannite, Manna sugar
Diuretic
Diagnostic for renal function

Mannitol is an osmotic diuretic, which is sometimes but rarely mentioned as an aid for drug penetration through the skin.

### N,N-Dimethylacetamide

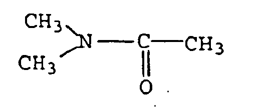

Vehicle for anti neoplastic agents
Industrial and pharmaceutical solvent

### Dimethylformamide (DMF)

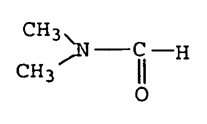

These compounds can be associated with toxicological adverse effects such as nausea, vomiting, loss of appetite, headache, and liver damage. Although DMF is not carcinogenic, it may increase absorption of heavy metal carcinogens through the skin.

### Preferred Inventive Compositions

The formulations of the present invention relate to the treatment of age spots, skin folds, diminished hair growth, and/or cellulites as well as other skin related disorders. These formulations, which are compositions comprising unique combinations of the following ingredients, exhibit novel activities, as described below.

Urea occurs naturally in the skin. When applied topically on the skin it develops a hydrating and keratolytic activity, in order to prepare the skin for the uptake of water soluble substances that are active against light/age spots and/or cellulites.

L-Carnitin (base or hydrochloride) is an essential cofactor of fatty acid metabolism and a carrier for acyl-function in the membrane of the Mitochondrium. Therefore, deficiencies in the subcutis structure in the fatty tissue of the subcutis that are collected in the corium as a second skin layer are regulated again to a normal metabolism and appearance of the skin area treated.

Tretinoin is a physiological metabolite of Vitamin A and is effective on gene expression via nuclear retinoid acid receptors (RAR). Tretinoin therefore mediates cellular growth and differentiation and regulates or repairs the code when damaged. Age-related light spots as well as cellulites certainly belong to this category of skin disorders that have their origins in damaged DNA or RNA chains.

DMSO plays an important role as it is rapidly absorbed through the skin and mucous membranes and enhances dermal absorption of many other chemicals.

Importantly, in the inventive formulations, DMSO, together with urea, and tretinoin (and optionally carnitin) transports the mixture of compounds rapidly (within minutes) through the skin layers to the cellular area where each component's single activity commences to develop. Preclinical studies show that age/light spots disappear in about a month's time when product is applied daily.

The same effects have been observed with cellulites, but with a somewhat gradual disappearance observed, as such pathological appearance in the skin layers usually develops gradually and slowly over the years. However, short preclinical trials already have shown a significant improvement and there is no known product on the market that improves cellulites significantly and visibly in just a few week's time.

A known, simple ointment has been chosen for inclusion in some embodiments of the inventive compositions. The hydrophilic nonioinic ointment exhibits suitable stability toward the other ingredients. The ointment, referred to as unguentum hydrophilicum non ionic, exists in the international pharmacopoea and is commercially available in any quantities.

Furthermore, lotion and spray formulations in accordance with the invention include a solvent comprising isopropyl alcohol and water, preferably, purified water.

Accordingly, novel compositions are provided, based on unique combinations of ingredients that together provide effective treatment of various skin disorders, when such combinations are included and administered in topical skin formulations.

In light of the preceding description and the following examples one of ordinary skill in the art can practice the invention to its fullest extent.

### EXAMPLE 1

### Composition suitable for the treatment of age related skin spots and skin folds in accordance with the invention

| **Component** | **Parts by weight** |
|---|---|
| Urea | 30.0 |
| L-Carnitin Base or Hydrochloride | --- |
| Tretinoin | 0.05 |
| Dimethylsulfoxide | 5.0 |
| Hydrophilic non ionic ointment | 65.0 |
| Total | 100.05 |

### EXAMPLE 2

### Composition suitable for the treatment of noninfectious cellulite in accordance with the invention

| **Component** | **Parts by weight** |
|---|---|
| Urea | 30.0 |
| L-Carnitin Base or Hydrochloride | 10.0 |
| Tretinoin | 0.05 |
| Dimethylsulfoxide | 5.0 |
| Hydrophilic non ionic ointment | 55.0 |
| Total | 100.05 |

### EXAMPLE 3

### A preferred hydrophilic non ionic ointment with components shown in parts by weight

| | |
|---|---|
| Polysorbate 60 | 5 |
| Cetylstearylalcohols | 10 |
| Glycerol 85% | 10 |
| White Vaseline | 25 |
| Water | 5 |
| | 100 |

### EXAMPLE 4

A preferred lotion or spray formulation in accordance with the invention, with components shown in parts by weight:

| | |
|---|---|
| Urea | 10.0 |
| L-Carnitin Base or Hydrochloride | - |
| Tretinoin | 0.05 |
| Dimethylsulfoxide | 10.0 |
| Solvent for lotion or spray | 80.0 |
| Total | 100.05 |

This formulation is particularly suitable for the treatment of skin spots and skin folds.

### EXAMPLE 5

A preferred lotion or spray formulation in accordance with the invention, with components shown in parts by weight:

| | |
|---|---|
| Urea | 10.0 |
| L-Carnitin Base or Hydrochloride | 10.0 |
| Tretinoin | 0.05 |
| Dimethylsulfoxide | 10.0 |
| Solvent for lotion or spray | 70.0 |
| Total | 100.05 |

This formulation is particularly suitable for the treatment of non-infectious cellulite.

### EXAMPLE 6

A preferred solvent mixture (for lotion or spray formulations) with the components shown in parts by volume:

| | |
|---|---|
| Isopropyl alcohol | 70.0 |
| (2-propanol, isopropanol) | |
| Aqua purificata | 30.0 |
| (Purified water) | |
| Total | 100.0 |

### EXAMPLE 7

In preclinical studies, compositions in accordance with the above examples were administered to human subjects exhibiting age spots and/or cellulites. Daily application of compositions in accordance with the present invention resulted in age spots disappearing in about 1 month time, and a reduction in the appearance of cellulites folds in a few week's time.

## Claims

1. A composition for treatment of a skin condition, said composition comprising 0.01-1 part by weight tretinoin and 1-45 parts by weight dimethylsulfoxide and further comprising at least one of 5-40 parts by weight urea and 5-15 parts by weight L-carnitine base or L-carnitine hydrochloride.

2. A composition as claimed in claim 1 comprising 20-40 parts by weight urea.

3. A composition as claimed in claim 1 or claim 2 wherein said composition is for topical administration.

4. A composition as claimed in any of claims 1 to 3 further comprising 45-90 parts by weight pharmaceutically or cosmetically acceptable carrier.

5. A composition as claimed in claim 4 comprising 45-75 parts by weight said carrier.

6. A composition as claimed in claim 4 or claim 5 wherein said carrier is hydrophilic and nonionic.

7. A composition as claimed in claim 6 wherein said carrier is an ointment.

8. A composition as claimed in claim 7 wherein said hydrophilic nonionic ointment comprises 1-10 parts by weight polysorbate 60, 5-15 parts by weight cetylstearylalcohols, 5-10 parts by weight glycerol, 20-30 parts per weight petroleum jelly and 1-10 parts by weight water.

9. A composition as claimed in claim 8 wherein said glycerol is glycerol 85%.

10. A composition as claimed in claim 7 comprising about 30 parts by weight urea, about 0.05 parts by weight tretinoin, about 5 parts by weight dimethylsulfoxide and about 65 parts by weight hydrophilic nonionic ointment.

11. A composition as claimed in claim 7 comprising about 30 parts by weight urea, about 0.05 parts by weight tretinoin, about 5 parts by weight dimethylsulfoxide and about 55 parts by weight hydrophilic nonionic ointment, and further comprising about 10 parts by weight L-carnitine base or L-carnitine hydrochloride.

12. A composition as claimed in any of claims 8 to 11 wherein said hydrophilic nonionic ointment comprises about 5 part by weight polysorbate 60, about 10 parts by weight cetylstearylalcohols, about 10 parts by weight glycerol 85%, about 25 parts by weight petroleum jelly and about 5 parts by weight water.

13. A composition as claimed in claim 4 or claim 5 wherein said carrier is a pharmaceutically or cosmetically acceptable solvent.

14. A composition as claimed in claim 13 wherein said solvent comprises isopropyl alcohol and water.

15. A composition as claimed in claim 14 wherein said solvent comprises about 70 parts per volume isopropyl alcohol and about 30 parts per volume water.

16. A composition as claimed in claim 15 comprising about 10 parts by weight urea, about 0.05 parts by weight tretinoin, about 10 parts by weight dimethylsulfoxide and about 80 parts by weight said solvent.

17. A composition as claimed in claim 15 comprising about 10 parts by weight urea, about 0.05 parts by weight tretinoin, about 10 parts by weight dimethylsulfoxide and about 70 parts by weight of said solvent, and further comprising about 10 parts by weight L-carnitine base or L-carnitine hydrochloride.

18. Use of tretinoin and dimethylsulfoxide in respective amounts of 0.01-1 part by weight and 1-45 parts by weight, optionally together with urea in an amount of 5-40 parts by weight and optionally further together with L-carnitine base or L-carnitine hydrochloride in an amount of 5-15 parts by weight, for the manufacture of a medicament for treatment of a skin condition which is an age spot, liver spot, photodamaged skin or a skin fold.

19. A method of cosmetically treating a skin condition comprising administering a composition as claimed in any of claims 1 to 17 to an area of skin condition to be treated.

20. A method as claimed in claim 19 wherein said skin condition is non-infection cellulite.

## Patentansprüche

1. Zusammensetzung zur Behandlung einer Hautkrankheit bzw. eines Hautzustandes mit 0,01 - 1 Gewichtsanteil Tretinoin und 1 - 45 Gewichtsanteilen Dimethylsulfoxid, und ferner mit wenigstens entweder 5 - 40 Gewichtsanteilen Harnstoff und 5 - 15 Gewichtsanteilen L-Carnitinbase oder L-Carnitinhydrochlorid.

2. Zusammensetzung wie in Anspruch 1 beansprucht, mit 20 - 40 Gewichtsanteilen Harnstoff.

3. Zusammensetzung wie in Anspruch 1 oder 2 beansprucht, wobei die Zusammensetzung zur topischen Gabe ist.

4. Zusammensetzung wie in einem der Ansprüche 1 bis 3 beansprucht, ferner mit 45 - 90 Gewichtsanteilen eines pharmazeutisch oder kosmetisch akzeptablen Trägers bzw. Carriers.

5. Zusammensetzung wie in Anspruch 4 beansprucht, mit 45 - 75 Gewichtsanteilen des Trägers.

6. Zusammensetzung wie in Anspruch 4 oder 5 beansprucht, wobei der Träger hydrophil und nichtionisch ist.

7. Zusammensetzung wie in Anspruch 6 beansprucht, wobei der Träger eine Salbe ist.

8. Zusammensetzung wie in Anspruch 7 beansprucht, wobei die hydrophile, nichtionische Salbe 1 - 10 Gewichtsanteile Polysorbat 60, 5 - 15 Gewichtsanteile Cetylstearylalkohole, 5 - 10 Gewichtsanteile Glycerin, 20 - 30 Gewichtsanteile Vaselin und 1 - 10 Gewichtsanteile Wasser enthält.

9. Zusammensetzung wie in Anspruch 8 beansprucht, wobei das Glycerin 85%iges Glycerin ist.

10. Zusammensetzung wie in Anspruch 7 beansprucht, mit 30 Gewichtsanteilen Harnstoff, etwa 0,05 Gewichtsanteilen Tretinoin, etwa 5 Gewichtsanteilen Dimethylsulfoxid und etwa 65 Gewichtsanteilen hydrophiler, nichtionischer Salbe.

11. Zusammensetzung wie in Anspruch 7 beansprucht, mit etwa 30 Gewichtsanteilen Harnstoff, etwa 0,05 Gewichtsanteilen Tretinoin, etwa 5 Gewichtsanteilen Dimethylsulfoxid und etwa 55 Gewichtsanteilen hydrophiler, nichtionischer Salbe, ferner mit etwa 10 Gewichtsanteilen L-Carnitinbase oder L-Carnitinhydrochlorid.

12. Zusammensetzung wie in einem der Ansprüche 8 bis 11 beansprucht, wobei die hydrophile, nichtionische Salbe etwa 5 Gewichtsanteile Polysorbat 60, etwa 10 Gewichtsanteile Cetylstearylalkohole, etwa 10 Gewichtsanteile 85%iges Glycerin, etwa 25 Gewichtsanteile Vaselin und etwa 5 Gewichtsanteile Wasser enthält.

13. Zusammensetzung wie in Anspruch 4 oder 5 beansprucht, wobei die Trägersubstanz ein pharmazeutisch oder kosmetische akzeptables Lösungsmittel ist.

14. Zusammensetzung wie in Anspruch 13 beansprucht, wobei das Lösungsmittel Isopropylalkohol und Wasser enthält.

15. Zusammensetzung wie in Anspruch 14 beansprucht, wobei das Lösungsmittel etwa 70 Volumenanteile Isopropylalkohol und etwa 30 Volumenanteile Wasser enthält.

16. Zusammensetzung wie in Anspruch 15 beansprucht, mit etwa 10 Gewichtsanteilen Harnstoff, etwa 0,05 Gewichtsanteilen Tretinoin, etwa 10 Gewichtsanteilen Dimethylsulfoxid und etwa 80 Gewichtsanteilen des Lösungsmittels.

17. Zusammensetzung wie in Anspruch 15 beansprucht mit etwa 10 Gewichtsanteilen Harnstoff, etwa 0,05 Gewichtsanteilen Tretinoin, etwa 10 Gewichtsanteilen Dimethylsulfoxid und etwa 70 Gewichtsanteilen des Lösungsmittels, ferner mit etwa 10 Gewichtsanteilen L-Carnitinbase oder L-Carnitinhydrochlorid.

18. Verwendung von Tretinoin und Dimethylsulfoxid in jeweiligen Anteilen von 0.01 - 1 Gewichtsanteil und 1 - 45 Gewichtsanteilen, optional zusammen mit Harnstoff in einer Menge von 5 - 40 Gewichtsanteilen und optional ferner zusammen mit L-Carnitinbase oder L-Carnitinhydrochlorid in einer Menge von 5 - 15 Gewichtsanteilen zur Herstellung eines Medikaments zur Behandlung einer Hautkrankheit bzw. eines Hautzustandes, die bzw. der ein Altersfleck, ein Leberfleck, photogeschädigte Haut oder eine Hautfalte ist.

19. Verfahren zur kosmetischen Behandlung einer Hautkrankheit umfassend die Gabe einer Zusammensetzung wie in einem der Ansprüche 1 bis 17 beansprucht auf eine Region der Hautkrankheit, die behandelt werden soll.

20. Verfahren wie in Anspruch 19 beansprucht, bei dem die Hautkrankheit eine nicht infektionsbedingte bzw. infektiöse Cellulite ist.

## Revendications

1. Composition pour le traitement d'un état de la peau, ladite composition comprenant de 0,01 à 1 partie en poids de trétinoïne et de 1 à 45 parties en poids de diméthylsulfoxyde, et comprenant en outre au moins une de 5 à 40 parties en poids d'urée et de 5 à 15 parties en poids de L-carnitine base ou de chlorhydrate de L-carnitine.

2. Composition selon la revendication 1, comprenant de 20 à 40 parties en poids d'urée.

3. Composition selon la revendication 1 ou la revendication 2, ladite composition étant destinée à une administration topique.

4. Composition selon l'une quelconque des revendications 1 à 3, comprenant en outre de 45 à 90 parties en poids d'un support pharmaceutiquement ou cosmétiquement acceptable.

5. Composition selon la revendication 4, comprenant de 45 à 75 parties en poids dudit support.

6. Composition selon la revendication 4 ou la revendication 5, dans laquelle ledit support est hydrophile et non ionique.

7. Composition selon la revendication 6, dans laquelle ledit support est un onguent.

8. Composition selon la revendication 7, dans laquelle ledit onguent hydrophile non ionique comprend de 1 à 10 parties en poids de polysorbate 60, de 5 à 15 parties en poids d'alcools cétylstéaryliques, de 5 à 10 parties en poids du glycérol, de 20 à 30 parties en poids de vaseline et de 1 à 10 parties en poids d'eau.

9. Composition selon la revendication 8, dans laquelle le glycérol est du glycérol à 85 %.

10. Composition selon la revendication 7, comprenant environ 30 parties en poids d'urée, environ 0,05 partie en poids de trétinoïne, environ 5 parties en poids de diméthylsulfoxyde et environ 65 parties en poids d'onguent hydrophile non ionique.

11. Composition selon la revendication 7 comprenant environ 30 parties en poids d'urée, environ 0,05 partie en poids de trétinoïne, environ 5 parties en poids de diméthylsulfoxyde et environ 55 parties en poids d'onguent hydrophile non ionique, et comprenant en outre environ 10 parties en poids de L-carnitine base ou de chlorhydrate de L-carnitine.

12. Composition selon l'une quelconque des revendications 8 à 11 dans laquelle ledit onguent hydrophile non ionique comprend environ 5 parties en poids de polysorbate 60, environ 10 parties en poids d'alcools cétylstéaryliques, environ 10 parties en poids de glycérol à 85 %, environ 25 parties en poids de vaseline et environ 5 parties en poids d'eau.

13. Composition selon la revendication 4 ou la revendication 5, dans laquelle ledit support est un solvant pharmaceutiquement ou cosmétiquement acceptable.

14. Composition selon la revendication 13, dans laquelle ledit solvant comprend de l'alcool isopropylique et de l'eau.

15. Composition selon la revendication 14, dans laquelle ledit solvant comprend environ 70 parties en volume d'alcool isopropylique et environ 30 parties en volume d'eau.

16. Composition selon la revendication 15, comprenant environ 10 parties en poids d'urée, environ 0,05 partie en poids de trétinoïne, environ 10 parties en poids de diméthylsulfoxyde et environ 80 parties en poids dudit solvant.

17. Composition selon la revendication 15, comprenant environ 10 parties en poids d'urée, environ 0,05 partie en poids de trétinoïne, environ 10 parties en poids de diméthylsulfoxyde et environ 70 parties en poids dudit solvant, et comprenant en outre environ 10 parties en poids de L-carnitine ou de chlorhydrate de L-carnitine.

18. Utilisation de trétinoïne et de diméthylsulfoxyde en des quantités respectives de 0,01 à 1 partie en poids et de 1 à 45 parties en poids, de préférence ensemble avec de l'urée en une quantité de 5 à 40 parties en poids et facultativement en outre conjointement avec de la L-carnitine base ou du chlorhydrate de L-carnitine en une quantité de 5 à 15 parties en poids, pour la fabrication d'un médicament pour le traitement d'un état de la peau consistant en une tache de vieillesse, tache du foie, de la peau abîmée par le soleil ou un pli de la peau.

19. Procédé pour traiter de façon cosmétique un état de la peau, comprenant l'application d'une composition selon l'une quelconque des revendications 1 à 17 à une zone de l'état de la peau à traiter.

20. Procédé selon la revendication 19, dans lequel ledit état de la peau est une cellulite non infectieuse.
